# EUROPEAN PATENT SPECIFICATION

(11) **EP 1 836 998 B1**
(45) Date of publication and mention of the grant of the patent: **24.02.2010**
(21) Application number: 07251205.6
(22) Date of filing: 21.03.2007
(51) Int. Cl.: A61F 2/84

(54) **Split sheath delivery system for self expanding stents**
Splitschleusenfreisetzungssystem für selbstexpandierende Stents
Système de mise en place d'une gaine fendue d'endoprothèse vasculaire auto-étendue

(30) Priority: 24.03.2006 US 388288
(43) Date of publication of application: 26.09.2007
(73) Proprietor: Cordis Corporation, Miami Lakes, FL 33014 (US)
(72) Inventor: Hoo, Bruce A., Miami, Florida 33173 (US); Johnson, Kirk L., Davie, Florida 33326 (US); Wilson, David, Denver, Colorado 80203 (US)
(74) Representative: Belcher, Simon James

(56) References cited:
- EP-A1- 0 732 087
- WO-A-01/24733
- US-A1- 2002 052 640
- US-A1- 2002 099 431
- US-B1- 6 447 540

## Description

The present invention generally relates to medical devices, particularly a self-expanding stent-sheath combination that facilitates delivery and deployment of the stent in a vessel.

A stent is a generally longitudinal tubular device formed of biocompatible material that is useful in the treatment of stenoses, strictures or aneurysms in blood vessels and other body vessels. Stents can be implanted within an unhealthy vessel to reinforce collapsing, partially occluded, weakened, or abnormally dilated sections of the vessel. Stents can be used after angioplasty of a blood vessel to prevent restenosis of the diseased vessel. While stents are most notably used in blood vessels, stents may also be implanted in other body vessels such as the urogenital tract and the bile duct. A stent may include a flexible configuration to allow it to be inserted through curved vessels. Furthermore, stents are often initially configured in a radially compressed state, by crimping or other techniques, to facilitate delivery and deployment in intraluminal catheter implantation.

Stents are used increasingly in place of or in addition to percutaneous transluminal angioplasty (PTA), which is administered for treatment of atherosclerosis, with the intention of reducing the need to repeatedly open an atherosclerotic artery. With passage through an atherosclerotic artery being substantially reduced, the area of stenosis often needs to be predilated with a small and low profile balloon (which is subsequently removed) in order to be able to position the stent delivery device for delivery of a self expandable stent at the desired location. Manipulation of the balloon and then the stent within the narrowed artery, which may have deposits of irregular and friable plaque, can cause thromboembolic complications. Friable plaque has the gross pathological appearance of degenerated, loose, fibroatheromatous debris. The dislodgment of a fragment of plaque can cause ischemic injury if it is not caught before it passes into critical downstream organs.

In the angioplasty procedure, a guide wire is inserted into the femoral artery and is passed into the diseased artery. A catheter having a balloon attached to its distal end is advanced along the guide wire to a point where the sclerotic lesions limit blood flow through the coronary artery. The balloon is then inflated, compressing the lesions radially outward against the wall of the artery and substantially increasing the size of its internal lumen to improve blood circulation through the artery.

A stent may be configured as an elongate structure, generally cylindrical in shape. The stent may exist in a first, pre-deployed state. The stent can be transformed into a second state, post delivery, with the stent, in the second state, having a substantially greater diameter than the diameter of the stent in the first state. The stent can be implanted in the vessel of a patient using the stent delivery system appropriate for the type of stent being delivered to the vessel.

While certain types of stents are self expanding, other stents, such as the Palmaz Blue® stent, available from Cordis Corporation of Miami Lakes, FL, USA, are expanded radially outward by the force imparted by an inflated angioplasty type balloon as the balloon pushes against the inner stent walls. An example of a self-expanding stent is the SMART® nitinol stent, a nickel titanium alloy stent also available from the Cordis Corporation.

Typically, a balloon expandable stent may be delivered to a site within the patient's vessel through a guide catheter. For this type stent, the stent is mounted on the balloon element of the angioplasty catheter without an external restraining sheath. The balloon is inflated to a pressure sufficient to produce a force that will radially expand the stent outward, which stretches the vessel and compresses the lesion to the inner wall of the vessel. On the other hand, a self-expanding stent possesses a spring force that causes the stent to expand following its implacement in the artery when a restraining sheath is retracted from the compressed stent. Alternatively, and by way of example, a self-expanding nitinol stent may be of the kind that expands when warmed above the martensitic transition temperature for the nitinol alloy (e.g., above 30° C.)

Where a balloon catheter is used to expand the stent, the balloon is subsequently removed from inside the stent and the catheter and other delivery apparatus is withdrawn. To determine if the process increased the lumen through the vessel, thereby improving blood flow, clinical examinations including angiography or ultrasonic morphological procedure are performed to determine if the stent emplacement procedure satisfactorily opened the diseased artery or vessel. These tests are often repeated periodically, as restenosis of the artery may sometimes occur.

Stents are akin to scaffoldings in their support of the passageway. Structurally, a stent may have two or more struts or wire support members connected together into a lattice-like frame. As indicated, stents may be in a compressed state prior to delivery and deployment, as compression facilitates insertion through small cavities. Stents can be delivered to the desired implantation site percutaneously through a catheter or similar transluminal device. With a lattice-like structure, a portion of the stent surface area is open, such openings defined by the struts that form the stent. Open spaces can be undesirable in that they can allow plaque from the lesion to fall through the stent and enter the blood stream.

US-A-2002/099431 discusses a constraining sheath for an endoprosthesis or stent. The constraining sheath comprises perforations that break when an angioplasty balloon, located under the stent and constraining the sheath, is inflated.

According to the present invention there is provided a stent assembly as defined in appended claim 1 and a stent delivery system as defined in appended claim 2. The present invention is directed to self-expanding stents within a sheath and a delivery system for same. The self-expanding stent-in-sheath arrangement facilitates the crimping of the stent and its delivery to the site of deployment in the vessel. The sheath can be constructed of a film that is wrapped around the stent and then fused to produce an encasing sheath. A region of compromised structural integrity is provided on the sheath, which region permits the sheath to rupture during expansion of the stent. The region of compromised structural integrity is a plurality of scores provided on the film and randomly arranged on the film. The outward pressure exerted upon the sheath by the expanding stent interior during the balloon inflation causes the sheath to burst at the weakened region. The ruptured sheath is then removed from the space between the stent and the vessel wall. Removal can be effected by fusing a portion of the sheath to the distal end of the stent delivery system. Thus, after rupture of the sheath at the time the stent is deployed, the ruptured sheath can be removed along with the removal of the SDS from the site of stent deployment.

The self-expanding stent-in-sheath arrangement described herein offers several advantages. Axial loading forces and the attendant friction associated with the loading of a self-expanding stent into a delivery device are diminished, if not eliminated, as the stent itself is never directly pushed during the loading or deployment. This is advantageous with regard to polymer coated drug eluting stents that have relatively higher friction surfaces, or with stents exhibiting higher axial flexibility, which do not respond as well to axial loading during crimping and loading. Further, accuracy of balloon deployment is believed to be improved in the case of nitinol stents, which exhibit as relatively high degree of elasticity.

Embodiments of the invention will now be described by way of example with reference to the accompanying drawings, in which:
Fig. 1 is a perspective view of a stent according to the present invention;
Fig. 2 is a perspective view of the manufacture of a stent according to the present invention at a time during its manufacture;
Fig. 3 is a perspective view of the manufacture of a stent according to the present invention at an earlier time during its manufacture;
Fig. 4 is a cross sectional view of a stent according to the present invention during its deployment in a vessel;
Fig. 5 is a cross sectional view of a stent according to the present invention during its deployment in a vessel at a time subsequent to what is depicted in Fig. 4.

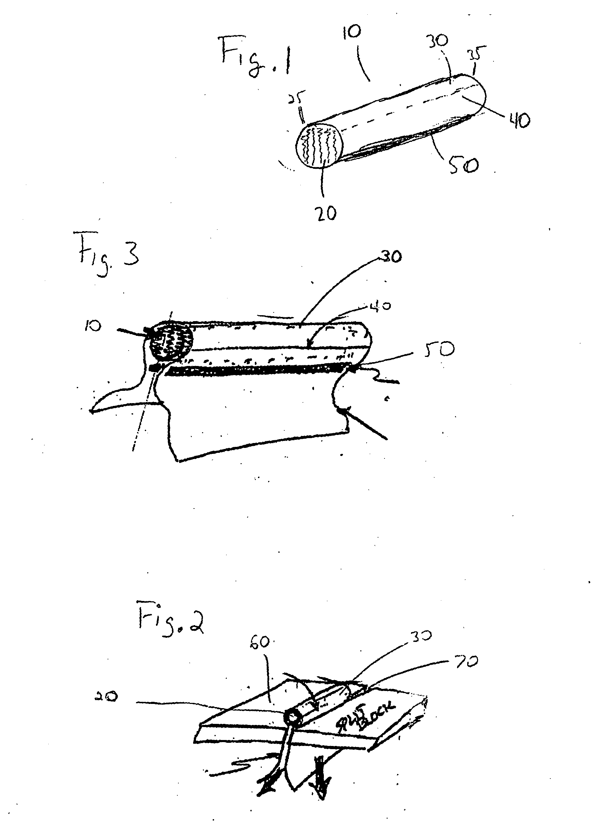

Figure 1 depicts an assembly 10 comprising a stent 20 wrapped within a sheath 30. The sheath can be constructed of a heat fusable film, such as polyamide (nylon, and polyethylene, polypropylene, to name but a few suitable materials. The sheath is provided with at least one region 40 of compromised structural integrity, rendering that region relatively weaker than the other regions of the sheath. As depicted in Fig. 1, to help understand the present invention, a score line extending parallel to the axis of the sheath comprises the relatively weaker region of compromised structural integrity. The sheath may be provided with a plurality of regions of relative weakness, which may be in the form of score lines. According to the invention, the scores or cuts may be randomly provided on the film.

Further, the fused region may itself constitute the region of compromised structural integrity. For example, fused regions of the sheath can be interspersed among non-fused regions. The non-fused regions would be relatively weaker than the nearby fused regions. Thus, upon application of the bursting force, the sheath would rupture at the non-fused regions.

Again any heat fusable material suited for use in the body of a person can be employed as the sheath. Particularly suited for use here are thermoplastic polymers such as high density polyethylene, nylon, polyesters, and other polyolefins, such as polypropylene and polybutylene.

The sheath and self-expanding stent arrangement can be manufactured as shown in Figs. 2 and 3. In Fig. 2, the stent, as yet uncrimped, is surrounded by the sheath and placed within a split block 60. The film 30 is wrapped around the stent and the film is drawn through a slot 70 in the split block. The film is pulled from the side of the block opposite the wrapped stent with force sufficient to crimp the stent. Heating elements in the split block (which are not shown) contact the thermoplastic film, raising the temperature to a level sufficient to fuse the film together. The excess film is then cut off to produce a crimped stent encased within the polymer sheath. The film is scored by a suitable scoring device in the patterns previously described, or by creating a relatively weak, rupturable fusion bond.

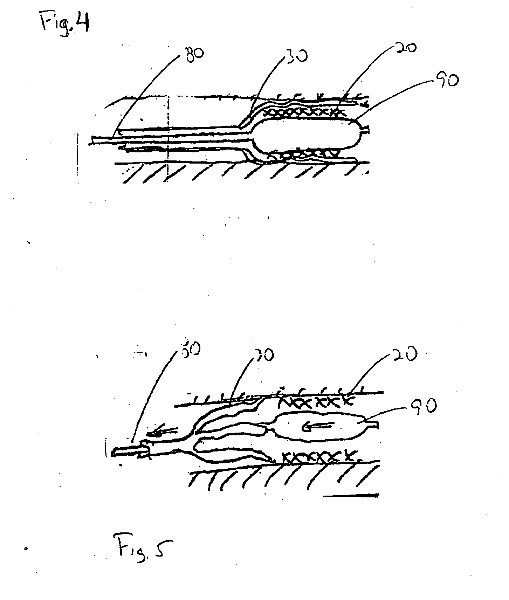

As shown in Fig. 4, the sheath 30 can then be fused to a lumen 95, such as by heat fusing. The lumen 95 is concentric with the balloon catheter shaft 80 of a stent delivery device, and the lumen 95 moves independently of the catheter shaft 80. The stent can be delivered to the site of deployment of the stent in accordance under procedures practiced in the art. Inflation of the balloon 90 attached distally to the balloon catheter shaft 80 applies a force to the interior walls of the stent 20, thereby causing the stent to expand. In turn, the expansion of the stent applies a force to the polymer sheath, eventually causing the polymer film to rupture. Once the film ruptures, the self expanding stent will expand freely to the maximum diameter permitted by the vessel, trapping the ruptured film between the stent and the vessel. Retraction of lumen 95 retracts the sheath as the balloon is held in place inside the stent. Once the ruptured sheath is removed from between the stent and the vessel, the stent is expanded to the desired degree, the balloon is deflated and the catheter shaft is withdrawn. See Fig. 5.

To facilitate the removal of the ruptured sheath from the site of stent deployment, it may advantageous to employ a sheath that includes one of more rails or wires of reinforcing material embedded therein, with the rails or wires aligned to extend longitudinally in the axial direction of the stent. In view of space constraints, such wires should be of a relatively small diameter, on the order of 0.13 mm (0.005 inch) or less. The sheaths may be constructed by sandwiching the wires between two plys of fusible film, which are then fused together to form the reinforced film suitable to encase the stent. It should be understood that the reinforcing wires should be constructed of a material that retains its structural integrity at temperatures above the temperature at which the heat fusion device operates.

## Claims

1. A stent assembly (10) comprising a crimped self-expanding stent (20) wrapped within a fused film (30), wherein the film (30) is provided with a rupturable region (40) of compromised structural integrity which comprises a plurality of scores provided on the film (30), and **characterised in that** scores are randomly arranged on the film (30).

2. A stent delivery system comprised of a distal end and a balloon catheter (80) in fluid communication with the distal end, and a stent assembly (10) as defined in claim 1, wherein a portion of the film (30) is further fused to the distal end of the stent delivery system.

3. The stent assembly of claim 1 or the stent delivery system of claim 2, wherein the film (30) is heat fused in at least one region, said region comprising the region (40) of compromised structural integrity.

4. The stent assembly or stent delivery system of claim 3, wherein said region comprising the region (40) of compromised structural integrity comprises fused portions interspersed among rupturable non-fused regions.

5. The stent assembly of claim 1 or the stent delivery system of claim 2, wherein the covering film (30) is a thermoplastic material selected from the group consisting of polyolefins, polyester, and nylon.

6. The stent assembly of claim 1 or the stent delivery system of claim 2, wherein the covering film (30) is further comprised of a reinforcing material.

## Patentansprüche

1. Stentanordnung (10), welche einen gecrimpten selbstexpandierenden Stent (20) umfasst, welcher innerhalb eines verschmolzenen Films (30) gewickelt ist, wobei der Film (30) mit einer aufbrechbaren Zone (40) von geschwächter Strukturintegrität versehen ist, welche mehrere Kerben umfasst, die auf dem Film (30) vorgesehen sind, und **dadurch gekennzeichnet, dass** Kerben willkürlich auf dem Film (30) angeordnet sind.

2. Stentzuführungssytem, welches ein distales Ende, einen Ballonkatheter (80) in Fluidverbindung mit dem distalen Ende und eine Stentanordnung (10), wie in Anspruch 1 definiert, umfasst, wobei ein Teilbereich des Films (30) weiterhin mit dem distalen Ende des Stentzuführungssystems verschmolzen ist.

3. Stentanordnung nach Anspruch 1 oder Stentzuführungssystem nach Anspruch 2, wobei der Film (30) in zumindest einer Zone wärmeverschmolzen ist, wobei die Zone die Zone (40) von geschwächter Strukturintegrität umfasst.

4. Stentanordnung oder Stentzuführungssystem nach Anspruch 3, wobei die Zone, welche die Zone (40) von geschwächter Strukturintegrität umfasst, verschmolzene Teilbereiche umfasst, welche zwischen aufbrechbaren nichtverschmolzenen Zonen verteilt sind.

5. Stentanordnung nach Anspruch 1 oder Stentzuführungssystem nach Anspruch 2, wobei der bedeckende Film (30) ein thermoplastisches Material ist, welches ausgewählt ist aus der Gruppe, welche aus Polyolefinen, Polyester und Nylon besteht.

6. Stentanordnung nach Anspruch 1 oder Stentzuführungssystem nach Anspruch 2, wobei der bedeckende Film (30) weiterhin ein Verstärkungsmaterial umfasst.

## Revendications

1. Ensemble de stent (10) comprenant un stent auto-expansible (2) serti, enveloppé à l'intérieur d'un film fondu (30), dans lequel le film (30) est prévu avec une région fracturable (40) d'intégrité structurelle compromise qui comprend une pluralité de marques prévues sur le film (30) et **caractérisé en ce que** les marques sont agencées de manière aléatoire sur le film (30).

2. Système de pose de stent composé d'une extrémité distale et d'un cathéter à ballonnet (80) en communication de fluide avec l'extrémité distale, et un ensemble de stent (10) selon la revendication 1, dans lequel une partie du film (30) est en outre fondue sur l'extrémité distale du système de pose de stent.

3. Ensemble de stent selon la revendication 1, ou système de pose de stent selon la revendication 2, dans lequel le film (30) est fondu à chaud dans au moins une région, ladite région comprenant la région (40) d'intégrité structurelle compromise.

4. Ensemble de stent ou système de pose de stent selon la revendication 3, dans lequel ladite région comprenant la région (40) d'intégrité structurelle compromise comprend des parties fondues dispersées parmi les régions non fondues fracturables.

5. Ensemble de stent selon la revendication 1, ou système de pose de stent selon la revendication 2, dans lequel le film de recouvrement (30) est un matériau thermoplastique choisi dans le groupe comprenant les polyoléfines, le polyester et le nylon.

6. Ensemble de stent selon la revendication 1 ou système de pose de stent selon la revendication 2, dans lequel le film de recouvrement (30) est en outre composé d'un matériau de renforcement.
